# EUROPEAN PATENT SPECIFICATION

(11) **EP 0 587 288 B1**
(45) Date of publication and mention of the grant of the patent: **29.12.1997**
(21) Application number: 93305736.6
(22) Date of filing: 21.07.1993
(51) Int. Cl.: A61K 7/48, A61K 7/06

(54) **Cosmetic composition containing a lipid and a hydroxy or ketocarboxylic acid**
Kosmetische Zusammensetzung, die einen Lipidstoff und eine Hydroxy - oder Ketofettsäure enthält
Composition cosmétique contenant un lipide et un acide hydroxy ou ketocarboxylique

(30) Priority: 24.07.1992 GB 9215738; 05.08.1992 GB 9216626
(43) Date of publication of application: 16.03.1994
(73) Proprietor: UNILEVER PLC, London EC4P 4BQ (GB); UNILEVER N.V., 3013 AL Rotterdam (NL)
(72) Inventor: Bowser, Paul Anthony, Unilever Research, Bebington, Wirral, Merseyside, L63 3JW (GB); Evenson, Alan, Unilever Research U.S.Inc., Edgewater, New Jersey 07020 (US); Rawlings, Anthony, Unilever Research U.S.Inc., Edgewater, New Jersey 07020 (US)
(74) Representative: Evans, Jacqueline Gail Victoria

(56) References cited:
- EP-A- 0 340 592
- EP-A- 0 474 270
- WO-A-88/06880
- WO-A-90/01323
- DE-A- 2 436 468
- US-A- 4 363 815

## Description

### FIELD OF THE INVENTION

The invention relates to the use of a cosmetic composition for topical application to skin comprising a lipid component, selected organic acids and a cosmetically acceptable vehicle for the treatment of dry skin conditions.

### BACKGROUND TO THE INVENTION AND PRIOR ART

It is generally understood that ceramides present within the intercellular lipid lamellae of the stratum corneum play an important role in the production and maintenance of the water permeability barrier of the skin. Ceramides, or substances closely related to them, have been widely disclosed as components of skin care compositions.

For example Unilever NV in EP 0 097 059 disclosed the vital role played by ω-linoleoyl ceramides in the water barrier of the skin and described the synthesis and application for skin care of such ω-substituted ceramides.

Also Kao Corporation, in GB 2 178 312 and GB 2 213 723, disclose the use of natural ceramides extracted from skin in topical products, and in EPO 227 994, they disclose synthetic analogues of ceramides.

These intercellular lipid lamellae are also known to contain other lipids such as sterols and fatty acids. It is believed that one of the causes of dry skin is a reduction in the amount of lipid contained within these intercellular lipid lamellae. It is therefore desirable to be able successfully to replace these depleted lipids via the topical route.

Lamellae, however, are not static structures but are dynamic with considerable mobility of individual lipids within each distinct layer. The relative mobility or fluidity of these layers is a factor which is fundamentally important to the ultimate state and condition of the skin.

Recent work within Unilever suggests that changes in membrane or bilayer fluidity could contribute to the etiology of skin conditions, such as dryness, dandruff, spots and pimples.

A two-fold problem therefore exists, firstly it is desirable to be able successfully to replace these depleted lipids and this involves devising a suitable method for delivering these lipids effectively to the lipid lamellae, and secondly it is also desirable to re-establish the required mobility of these layers.

We have now solved this problem by a cosmetic composition for topical application to skin hair or nails comprising:
(i) selected lipid components
(ii) selected organic acids
(iii) a suitable cosmetic vehicle
for the treatment of dry skin conditions.

Organic acids such as hydroxy acids have been previously disclosed as beneficial for a wide range of skin disorders. Furthermore EP 273 202 (Van Scott) discloses the use of specific hydroxycarboxylic acids to enhance the percutaneous penetration or therapeutic efficacy of cosmetic and pharmaceutical agents.

EP 474 270 discloses a cosmetic emulsion suitable for topical application to the lips that comprises in addition to oil and water, an emulsifier system comprising phospholipid and a second emulsifier having a melting point of from -20°C to 80°C.

WO 88/06880 describes a cosmetic base composition exhibiting therapeutic properties comprising an acyl fatty acid lactylate ester or alkali metal salt thereof, a sucrose fatty acid ester and a solvent.

Surprisingly we have now shown that specific organic acids considerably effect the mobility or phase behaviour of the lipid lamellae of the stratum corneum. Furthermore we have shown that the use of a cosmetic composition comprising both hydroxy acids and specific lipid components is able to both deliver the lipid components to the lipid lamellae and re-establish the fluidity of these lamellae hence alleviating dry skin conditions.

### SUMMARY OF THE INVENTION

The invention accordingly provides for the use of a composition for topical application to skin, hair or nails comprising:
(i) a lipid component chosen from ceramides, pseudoceramides, and mixtures thereof;
(ii) an organic acid component chosen from hydroxy carboxylic acids, keto carboxylic acids and mixtures thereof; and
(iii) a cosmetically acceptable vehicle.
for the treatment and/or alleviation of dry skin conditions.

### DISCLOSURE OF THE INVENTION

### The lipid component

The lipid component is chosen from ceramides, pseudoceramides and mixtures thereof.

### Ceramides

Ceramides are preferably selected from ceramides having the general structure (1) 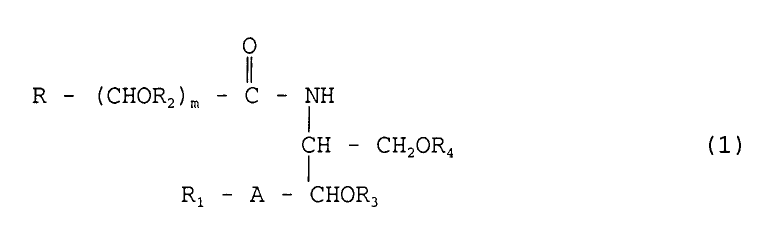 where A represents - CH₂ -; - CHOR₅ -; - CH=CH - or - CHOY -
R represents a linear or branched saturated or unsaturated, hydroxylated or non-hydroxylated aliphatic hydrocarbon group having from 1 to 49 carbon atoms or a subgroup (2).

   Y - O - (CₐH_{b}) - (2)
R₁ represents a linear or branched, saturated or unsaturated, hydroxylated or non-hydroxylated aliphatic hydrocarbon group having from 8 to 28 carbon atoms;
R₂, R₃ and R₅ individually represent H, a phosphate residue or a sulphate residue;
R₄ represents H, a phosphate residue, a sulphate residue or a sugar residue;
a is an integer of from 7 to 49
b is an integer of from 10 to 98
m is 0 or 1
Y represents H or a residue of a C₁₄₋₂₂ fatty acid having the general structure (3) 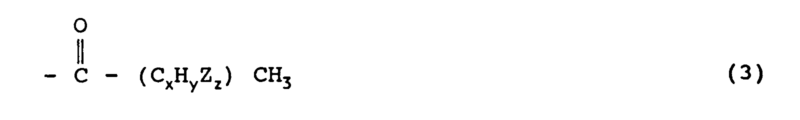 where
Z is - OH or an epoxy oxygen
x is an integer of from 12 to 20
y is an integer of from 20 to 40
   and
z is O or an integer of from 1 to 4

Ceramides having the general structure (1) are naturally occurring and can be isolated from a suitable plant source or from animal tissue such as pig skin or neural tissue. Ceramides can also be synthesised.

Particular preferred examples of ceramides are ceramide-1 and ceramide-2.

### Pseudoceramides

Pseudoceramides are preferably selected from pseudoceramides (ie synthetic ceramide like structures) having the general structure (4): 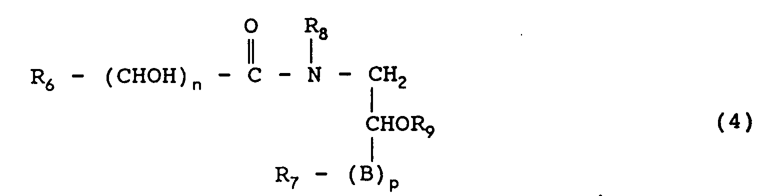 where B represents - OCH₂ - or CHOH.

R₆ represents a linear or branched, saturated or unsaturated, hydroxylated or non-hydroxylated aliphatic hydrocarbon group having from 1 to 49 carbon atoms or the subgroup (2).

R₇ represents a linear or branched, saturated or unsaturated, hydroxylated or non-hydroxylated hydrocarbon group having from 8 to 28 carbon atoms.

R₈ represents H, or a subgroup - (CH₂)_{c}COOH, where c is an integer of from 1 to 6, or a subgroup having the structure (5). 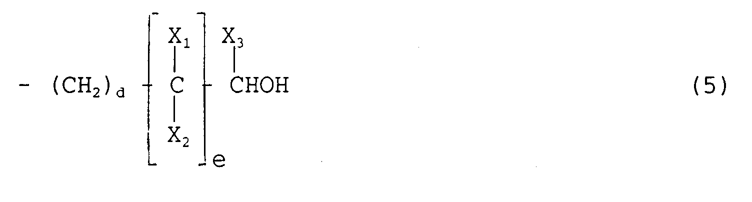 where X₁, X₂ and X₃ each individually represent H, a C₁₋₅ alkyl or a C₁₋₅ hydroxyalkyl;
d is 0 or an integer of from 1 to 4
e is 0 or 1
n is 0 or 1
   and
p is 0 or 1;
R₉ represents H, a phosphate residue, a sulphate residue or a sugar residue.

The composition may further comprise polyol fatty acid polyesters, phospholipids, galactosyldiacyl glycerol, glycosphingolipids and succinic acid derivatives.

### Polyol fatty acid polyesters

Polyol fatty acid polyesters are fatty acid polyesters derived from any aliphatic or aromatic polyol which has at least 4 free hydroxyl groups, of which at least 60% of these free hydroxyl groups are then esterified with one or more fatty acids having from 8 to 22 carbon atoms.

The polyol from which the polyol fatty acid polyesters are derived are preferably chosen from sugar polyols, which comprise mono-, di- and polysaccharides.

Preferred examples of monosaccharide sugar polyols include:
Pentose sugar polyols such as D-ribose, D-arabinose, D-xylose, D-lyxose, D-ribulose and D-xylulose.
Hexose sugar polyols such as D-allose, D-altrose, D-glucose, D-mannose, D-gulose, D-idose, D-galactose, D-talose, D-fructose, D-sorbose and D-tagatose.
Heptose sugar polyols, such as D-mannoheptulose and D-sedoheptulose.

The polyol from which the polyol fatty acid polyester is derived can also be chosen from:
disaccharides such as maltose, lactose, cellobiose, sucrose, trehalose, gentiobiose, melibiose and primeverose;
tri-saccharides, such as gentianose and raffinose;
sugar alcohols such as D-mannitol, D-sorbitol, D-ribitol, D-erithritol, D-lactitol and D-xylitol; and
derivatives of sugars such as α-methyl glucoside and inositol.

The preferred sugar polyol is sucrose.

The fatty acids which are employed to form the polyol fatty acid polyesters can be individual free fatty acids having from 8 to 22 carbon atoms in the fatty acid molecule.

These fatty acids can be saturated or unsaturated, linear or branched chain fatty acids.

A preferred source of fatty acids for forming the polyol fatty acid polyesters are naturally occurring fats and oils which provide a source of a blend of fatty acids residues, whose choice can vary widely the physical and chemical properties of the polyol fatty acid polyesters obtained therefrom.

These naturally occurring fats and oils can be used as obtained from nature or following full or partial hydrogenation, interesterification, transesterification or fractionation.

Suitable natural sources of these fatty acid residues may be of animal, marine or vegetable origin, such as tallow, lanolin oil, cod liver oil, halibut liver oil, other fish oils, coconut oil, palmkernel oil, palm oil, butter fat, soyabean oil, safflower oil, cotton seed oil, rapeseed oil, poppy seed oil, corn oil, sunflower oil, ground nut oil and mixtures thereof. Preferred fatty acid sources are palm oils, partially hydrogenated palm oils, palmkernel oils, optionally partially hydrogenated soya bean oils and partially hydrogenated fish oils.

By employing a mixture of fatty acids, or one or more naturally occurring oils such as those exemplified above, when synthesising the polyol fatty acid polyester, it is possible to provide polyol fatty acid polyesters in which a mixture of ester groups is present on a single polyol molecule. In this way it is possible to vary the melting characteristics of the polyol fatty acid polyester so formed as desired.

Particularly preferred polyol fatty acid polyesters are sucrose fatty acid polyesters where the ester is derived from lauric acid or natural oils, such as palm oil, palm kernal oil, soyabean oil, coconut oil, fish oil and mixtures thereof.

### Succinic acid derivatives

Suitable succinic acid derviatives have the general structure (6) 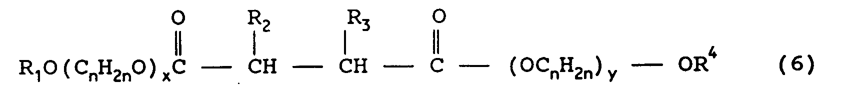 in which R₁ represents an alkyl, alkenyl, mono- or dihydroxyalkyl or hydroxyalkenyl group having from 6-22 carbon atoms;
R₂ and R₃ individually represent H or an alkyl or alkenyl group having from 12 to 22 carbon atoms; providing that when R₂ is H, R₃ is an alkyl or alkenyl group and when R₃ is H, R₂ is an alkyl or alkenyl group;
R₄ represents hydrogen, an alkyl, alkenyl, mono- or dihydroxyalkyl or hydroxyalkenyl group having from 6 to 22 carbon atoms or the group (7): 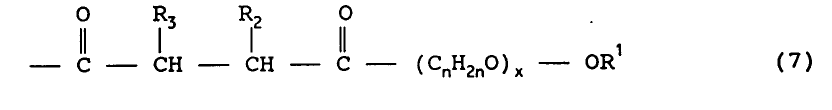
n is an integer of from 2 to 3
x and y are average degrees of alkoxylation, namely x is from 0 to 20 and y is from 1 to 20; and

In structure (6), the group R₄ preferably represents H, while the group R₁ preferably represents an alkyl group having from 16 to 22 carbon atoms and most preferably from 20 to 22 carbon atoms.

Also with reference to structure (6), n and m are preferably 2 and (x+y) is preferably from 1 to 20.

Specific examples of these succinic acid derivatives are those having the structures (8) to (12):

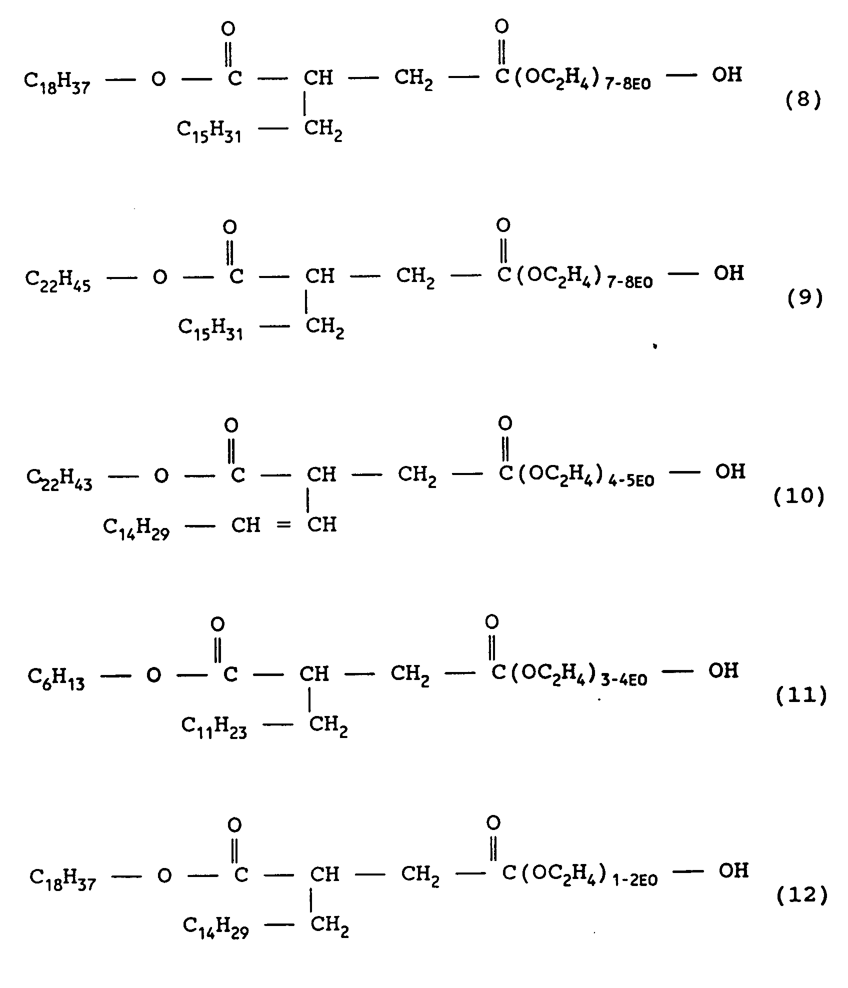

The composition according to the invention may contain more than one succinic acid derivative having the structure (6). In particular, it may be advantageous to incorporate at least two derivatives wherein for one derivative at least one of R₁ and R₂/R₃ is an alkenyl or hydroxyalkenyl group and for the second derivative both R₁ and R₂/R₃ are alkyl or hydroxyalkyl groups. A particularly preferred example of such a composition is one comprising structure (8) and structure (10).

Suitable methods of synthesising these succinic acid serivatives are disclosed in the GB patent application number 9223578.7.

Preferably the lipid components (i) is selected from ceramides having the structure (1), pseudoceramides having the structure (4), and mixtures thereof.

Preferably the amount of the lipid component (i) present in the composition according to the invention is from 0.00001 to 50%, more preferably from 0.001 to 20% and most preferably from 0.1 to 10% by weight.

### The organic acid component

The organic acid component is chosen from hydroxy carboxylic acids and keto carboxylic acids, and mixtures thereof.

The hydroxy acid can be chosen from α-hydroxy acids, β-hydroxyacids, other hydroxycarboxylic acids and mixtures thereof.

Preferably the hydroxy acid (ii) is chosen from α-hydroxy acids having the general structure (13): 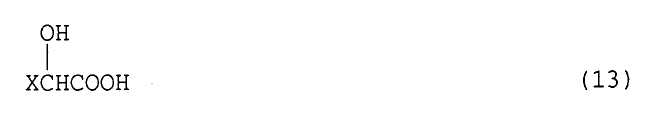 where
X is H - or CH₃(C_{f}H_{g})ₕ -
f is an integer of from 1 to 27,
g is an integer of from 2 to 54, and
h is 0 or 1.
hydroxymonocarboxylic acids having the general structure (14):

R₁₀(CR₁₁OH)_{q}(CH₂)ᵣCOOH (14)

where R₁₀ and R₁₁ are individually H or an alkyl, aralkyl or aryl group of saturated, unsaturated, branched or unbranched chain or cyclic form having from 1 to 25 carbon atoms
where
q is an integer of from 1 to 9, and
r is 0 or an integer of from 1 to 23;
hydroxydicarboxylic acids having the general structure (15) 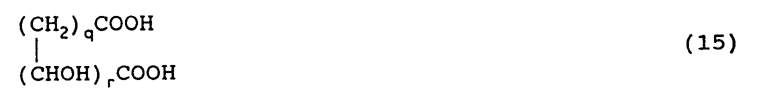 where
q is an integer of from 1 to 9, and
r is 0 or an integer of from 1 to 23;
hydroxycarboxylic acids having the general structure (16)

R₁₂(OH)_{q}(COOH)ₛ (16)

where q and s are individually an integer of from 1 to 9;
and R₁₂ is H, alkyl, aralkyl or aryl group of saturated or unsaturated, straight or branched chain or cyclic form having from 1 to 25 carbon atoms;
and mixtures thereof.

More preferably the organic acid compound (ii) is an α-hydroxy acid having the general structure (13). Even more preferably the hydroxy acid is chosen from 2-hydroxyoctanoic acid, lactic acid, and glycolic acid, and mixtures thereof.

The keto acids can be chosen from α-keto acids, β-keto acids and mixtures thereof.

A particularly preferred α-keto acid is 2-keto octanoic acid.

Preferably the amount of the organic acid component (ii) present in the composition according to the invention is from 0.01 to 20%, more preferably from 0.05 to 10% and most preferably from 0.1 to 2% by weight.

### Sterols

Preferably the cosmetic composition additionally comprises sterols.

Sterols are preferably selected from cholesterol, pro-vitamin D₃, campesterol, stigmastanol, stigmasterol, 5-dihydrocholesterol, α-spinasterol, palysterol, clionasterol, γ-sitosterol, stigmastenol, sargasterol, avenasterol, ergostanol, sitosterol, corbisterol, chondrillasterol, poriferasterol, haliclonaseterol, neospongosterol, fucosterol, aptostanol, Ergostadienol, ergosterol, 22-dihydroergosterol, brassicasterol, 24-methylenecholesterol, 5-dihydroergosterol, dehydroergosterol, 14-dehydroergosterol, 24-dehydroergosterol, fungisterol, cholestanol, coprostanol, Zymosterol, 7-hetocholesterol, Lathosterol, 22-dehydrocholesterol, 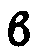 -sitosterol, cholestatrien-3 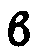 -01, coprostanol, cholestanol, ergosterol, 7-dehydrocholesterol, 24-dehydrocholest-adione-3 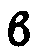 -01, equilenin, equilin, estrone, 17 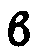 -estradiol, Androst-4-ene-3 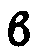 , 17 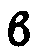 -diol, dehydroepiandrosterone and mixtures thereof.

More preferably the sterol is selected from cholesterol, any plant sterol, pro-vitamin D₃, 5-dihydrocholesterol and mixtures thereof.

Even more preferably the sterol is cholesterol.

### Fatty acids

Preferably the cosmetic composition additionally comprises fatty acids.

The fatty acids are preferably essential fatty acids chosen from linoleic acid, γ-linolenic acid, homo-γ-linolenic acid, columbinic acid, eicosa-(n-6,9,13)-trienoic acid, arachidonic acid, α-linolenic acid, timnodonic acid, hexaenoic acid and mixtures thereof.

Non-essential fatty acids can also be employed in addition to or in place of essential fatty acids, examples of which are chosen from myristic, palmitic, stearic and isostearic acids, and mixtures thereof.

Most preferably the cosmetic composition additionally comprises both fatty acid and sterol.

### The Cosmetically Acceptable Vehicle

The composition according to the invention also comprises a cosmetically acceptable aqueous or non-aqueous vehicle to act as a dilutant, dispersant or carrier for the lipid component (i) and hydroxy acid (ii) in the composition, so as to facilitate its distribution when the composition is applied to the skin, hair and/or nails.

Vehicles other than water can include liquid or solid emollients, solvents, humectants, thickeners and powders. Examples of each of these types of vehicle, which can be used singly or as mixtures of one or more vehicles, are as follows:
Emollients, such as stearyl alcohol, glyceryl monoricinoleate, glyceryl monostearate, mink oil, cetyl alcohol, isoproopyl isostearatae, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, volatile or non-volatile silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, passion flower oil, avocado oil, olive oil, sesame seed oil, coconut oil, arachis oil, castor oil, acetylated lanolin alcohols, petroleum jelly, mineral oil, butyl myristate, isostearic acid, palmitatic acid, isopropyl linoleate, lauryl lactate, myristyl lactate, decyl oleate, myristyl myristate;
Propellants, such as air, propane, butane, isobutane, dimethyl ether, carbon dioxide, nitrous oxide;
Solvents, such as ethyl alcohol, methylene chloride, isopropanol, acetone, squalane, squalene, ethylene glycol monoethyl ether, diethylene glycol monobutyl ether, diethylene glycol monoethyl ether, polyethylene glycol, dimethyl sulphoxide, dimethyl formamide, butylene glycol, tetrahydrofuran;
Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the emulsion, and can, in the absence of other cosmetic adjuncts, form the balance of the composition.

### OPTIONAL SKIN BENEFIT MATERIALS AND COSMETIC ADJUNCTS

Although the composition according to the invention can be aqueous or non-aqueous, a particularly convenient form is an emulsion, in which case an oil or oily material will normally be present, together with an emulsifier to provide a water-in-oil emulsion, an oil-in-water emulsion, or a complex emulsion, depending largely on the average hydrophilic-lyophilic balance (HLB) of the emulsifier employed.

### Oil or oily material

The composition according to the invention can optionally comprise one or more oils or other materials having the properties of an oil.

Examples of suitable oils include mineral oil and vegetable oils, and oil materials, such as those already proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

### Emulsifier

The composition according to the invention can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, and the average HLB value.

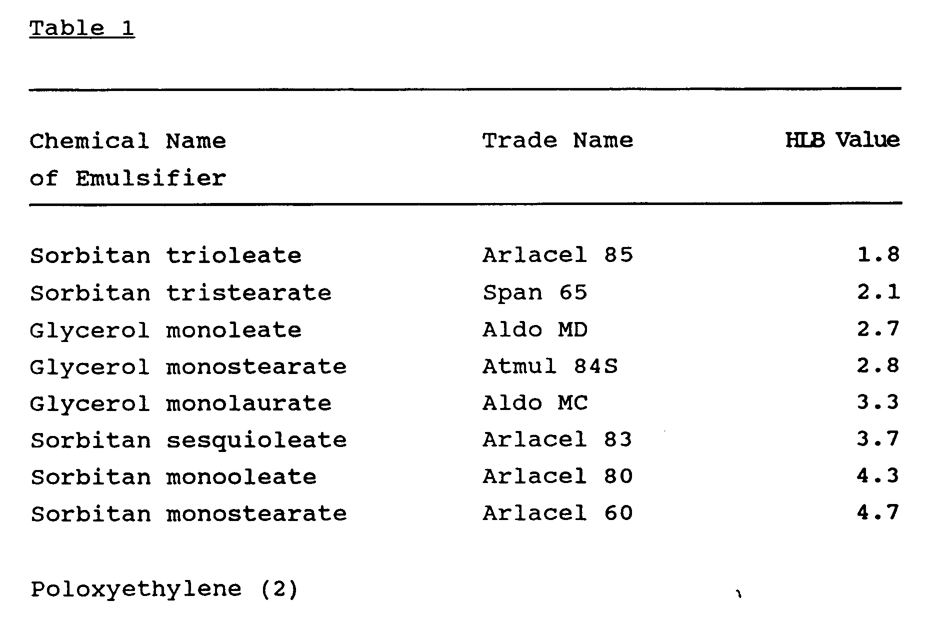

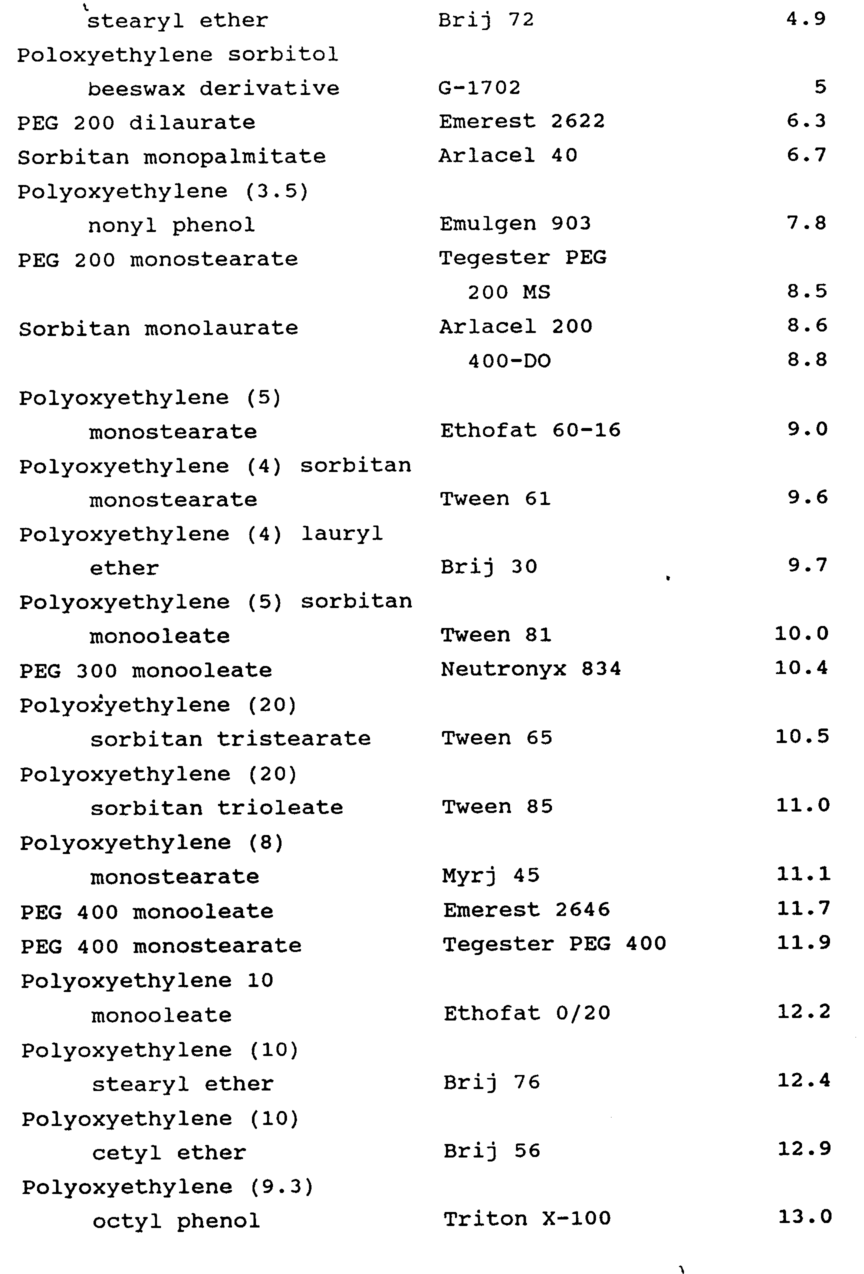

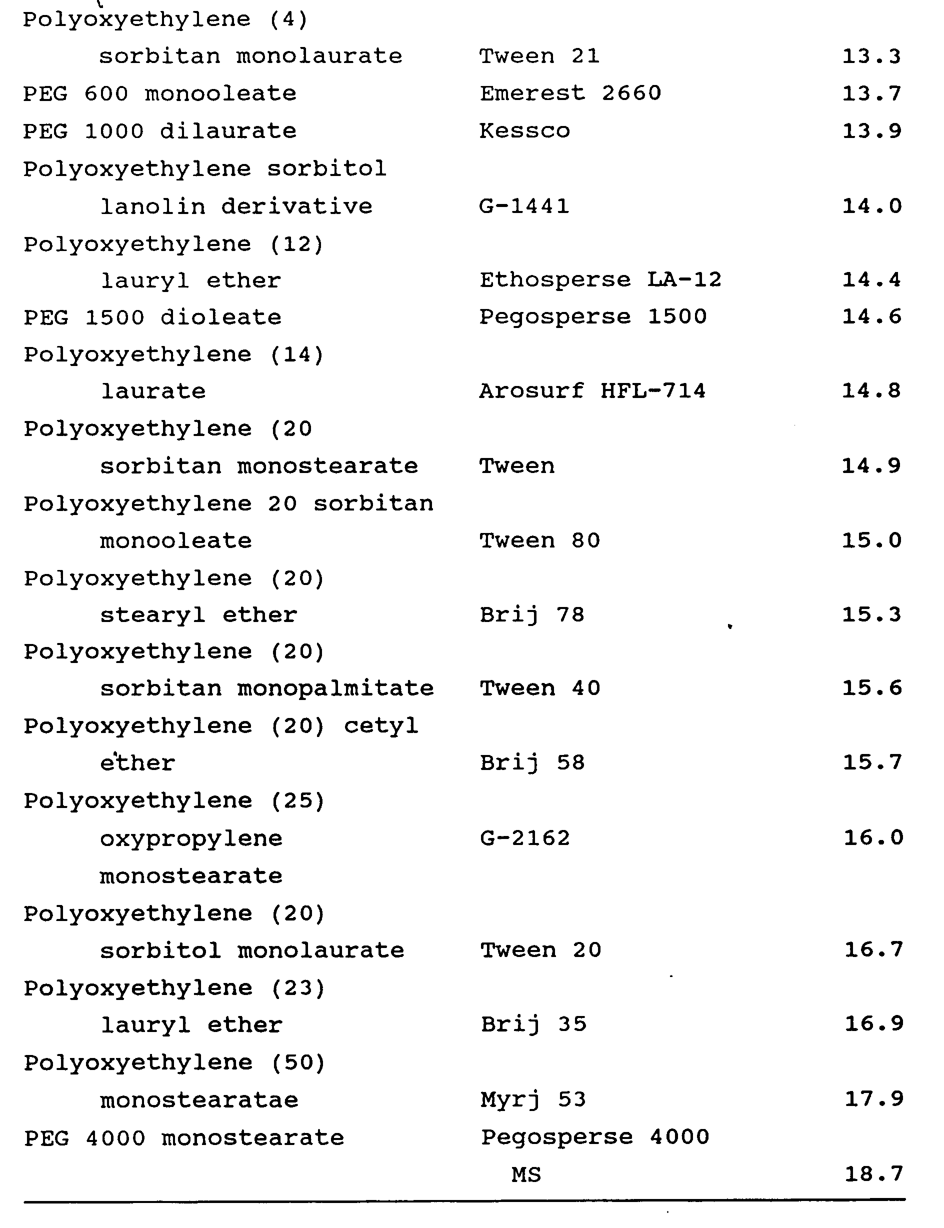

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, to be incorporated in the composition of the invention, when appropriate is from 1 to 50%, preferably from 2 to 20% and most preferably from 2 to 10% by weight of the composition.

### Water

Although the composition of the invention can be anhydrous, it can also comprise water, usually up to 98%, preferably from 5 to 80% by volume.

### Silicone Surfactant

The composition of the invention can also optionally comprise a high molecular weight silicone surfactant which can also act as an emulsifier, in place of or in addition to the optional emulsifier(s) already mentioned.

The silicone surfactant is a high molecular weight polymer of dimethyl polysiloxane with polyoxyethylene and/or polyoxypropylene side chains having a molecular weight of from 10,000 to 50,000 and having the structure: 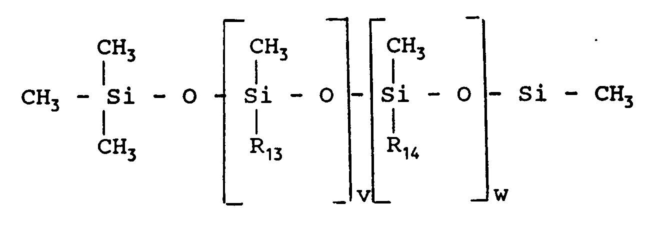 where the groups R₁₃ and R₁₄ are each chosen from -H, C₁₋₁₈ alkyl and 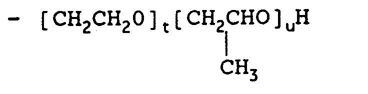
t has a value of from 9 to 115,
u has a value of from 0 to 50,
v has a value of from 133 to 673,
w has a value of from 25 to 0.25.

Preferably, the dimethyl polysiloxane polymer is one in which:
t has a value of from 10 to 114
u has a value of from 0 to 49
v has a value of from 388 to 402
w has a value of from 15 to 0.75
one of groups R₁₃ and R₁₄ being lauryl, and the other having a molecular weight of from 1000 to 5000.

A particularly preferred dimethyl polysiloxane polymer is one in which:
t has the value 14
u has the value 13
v has the value 249
w has the value 1.25

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2--5200, also available from Dow Corning.

The amount of silicone surfactant, when present in the composition will normally be up to 25%, preferably from 0.5 to 15% by weight of the emulsion.

### Other Adjuncts

Examples of conventional adjuncts which can optionally be employed include preservatives, such as para-hydroxy benzoate esters; antioxidants, such as α-tocopherol, humectants, such as glycerol, sorbitol, 2-pyrrolidone-5-carboxylate, dibutylphthalate, gelatin, polyethylene, glycol, preferably PEG 200-600; buffers, such as lactic acid together with a base such as triethanolamine or sodium hydroxide; surfactants, such as glycerol ethers; waxes, such as beeswax, ozokerite wax, paraffin wax, plant extracts, such as Aloe vera, cornflower, witch hazel, elderflower, cucumber; thickeners; activity enhancers; colourants; perfumes; and sunscreen materials such as ultrafine titanium dioxide and organic sunscreens such as p-aminobenzoic acid and esters thereof, ethylhexyl p-methoxycinnamate, 2-ethoxyethyl p-methoxycinnamate and butyl methoxydibenzoylmethane; and skin benefit agents, such as retinoic acid, retinol, retinol esters; anti-inflammatory agents, such as salicylic acid; skin whiteners, such as arbutin and mixtures thereof.

Cosmetic adjuncts can form the balance of the composition.

### Use of the Composition

The composition according to the invention is intended primarily as a product for topical application to human skin for the treatment and/or alleviation of dry skin conditions, especially as an agent for reducing the permeability of water through the skin, particularly when the skin is dry or damaged, in order to reduce moisture loss and generally to enhance the quality of skin. The composition can also be applied to hair and nails.

In use, a small quantity of the composition, for example from 0.2 to 5 ml, is applied to exposed areas of the skin, from a suitable container or applicator and, if necessary, it is then spread over and/or rubbed into the skin using the hand or fingers or a suitable device.

### METHOD OF TREATMENT

The invention also provides a method for the treatment and /or alleviation of skin dryness conditions which method includes topically applying to the skin a cosmetic composition which comprises:
( i) a lipid component chosen from ceramides, pseudoceramides, and mixtures thereof;
( ii) an organic acid component chosen from hydroxycarboxylic acids, keto carboxylic acids, and mixtures thereof; and
(iii) a cosmetically acceptable vehicle.

### PRODUCT FORM AND PACKAGING

The topical skin, hair or nail treatment composition of the invention can be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. Alternative product forms include liquids, microemulsions, a sera, mousses and gels.

The composition can be packaged in a suitable container to suit its viscosity and intended use by the consumer. For example, a lotion or fluid cream, or the alternative product forms can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

The invention accordingly also provides a closed container containing a cosmetically acceptable composition as herein defined.

### EXAMPLES

The invention is illustrated by the following examples:

### Example 1

This example illustrates a suncare cream:

| Ingredient | % w/w |
|---|---|
| 2-hydroxy-n-octanoic acid | 1 |
| Ceramide-1 | 0.01 |
| Silicone oil 200 cts | 7.5 |
| Glycerylmonostearate | 3 |
| Cetosteryl alcohol | 1.6 |
| Polyoxyethylene-(20)-cetyl alcohol | 1.4 |
| Xanthan gum | 0.5 |
| Parsol 1789 | 1.5 |
| Octyl methoxycinnate (PARSOL MCX) | 7 |
| Perfume | qs |
| Colour | qs |
| Water | to 100 |

### Example 2

This example illustrates a non-aqueous skincare composition:

| | |
|---|---|
| Ingredient | % w/w |
| Silicone gum SE-30¹ | 10 |
| Silicone fluid 345² | 20 |
| Silicone fluid 344³ | 55.79 |
| Squalene | 10 |
| Ceramides | 0.01 |
| Linoleic acid | 0.01 |
| Cholesterol | 0.03 |
| 2-hydroxy-n-octanoic acid | 0.7 |
| Vitamin A palmitate | 0.5 |
| Vitamin E linoleate | 0.5 |
| Herbal oil | 0.5 |
| Ethanol | 2 |

| | |
|---|---|
| ¹ A dimethyl silicone polymer having a molecular weight of at least 50,000 and a viscosity of at least 10,000 centistokes at 25°C, available from GEC | |
| ² Dimethyl siloxane cyclic pentamer, available from Dow Corning Corp | |
| ³ Dimethyl siloxane tetramer, available from Dow Corning Corp | |

### Example 3

This example illustrates a suntan lotion:

| | |
|---|---|
| Ingredient | % w/w |
| 2-keto octanoic acid | 0.2 |
| Ceramide-2 | 0.02 |
| Acetulan (cetyl acetate and acetylated lanolin alcohol) | 4 |
| Propylene glycol | 3 |
| Stearic acid | 2 |
| Dow Corning 556 fluid (phenyl dimethicone) | 1 |
| Veegum (modified magnesium aluminium silicate) | 1 |
| Cetyl alcohol | 0.5 |
| Triethanolamine | 0.5 |
| Octyl methoxycinnamate | 1 |
| Oxybenzone | 1 |
| Preservatives | qs |
| Water | to 100 |

### Example 4: Comparative Examples A-E

### In vivo efficacy test

To establish effectiveness in vivo of lipid and α-hydroxy acid combinations, a dry flaky skin condition was first induced in subjects with soap washing over a period of one week in the winter season. When dryness had been produced, subjects were treated with lotions containing lipids and α-hydroxy acids twice daily. Each day the subjects skin condition was assessed by visual grading of the skin by expert assessors, on a scale of 1 to 5 of increasing dryness. The efficacy of the lipid plus α-hydroxy acid mixture was demonstrated by improved recovery of the skin condition compared with lotions containing only lipid and lotions containing only α-hydroxy acids. This is represented by percentage improvement where 0% means no recovery and 100% means full recovery. For each of the treatment groups 10 subjects participated. Results are shown in Table 1.

### Example

- 4: 4% lipid component [ceramide II, cholesterol and stearic acid (1:2:1 w/w)] 1% glycerol, 8% glycolic acid
- E: 4% lipid component [phospholipid, cholesterol, stearic acid (1:2:1 w/w)] 0.7% hydroxycaprylic acid
- A: 8% glycolic acid only
- B: 4% lipid component only (ceramide II, cholesterol, stearic acid (1:2:1 w/w))
- C: 8% hydroxycaprylic acid only
- D: 4% lipid component only (phospholipid, cholesterol and stearic acid (1:2:1 w/w))

**Table 1**

| EXAMPLE | % Improvement for No of Days of Treatment | | |
|---|---|---|---|
| | 1 | 3 | 6 |
| 4 | 53.8 | 66.5 | 66.6 |
| A | 6.25 | 18.8 | 18.8 |
| B | 25.0 | 44.8 | 49.0 |
| E | 17.4 | 34.0 | 52.3 |
| C | 6.7 | 0 | 0 |
| D | 0 | 12.5 | 25.0 |

## Claims

1. The cosmetic use of a composition for topical application to skin, hair or nails comprising:
(i) a lipid component chosen from ceramides, pseudoceramides, and mixtures thereof;
(ii) an organic acid component chosen from hydroxy-carboxylic acids, keto carboxylic acids, and mixtures thereof; and
(iii) a cosmetically acceptable vehicle;
for the treatment and/or alleviation of dry skin conditions.

2. The cosmetic use of a composition according to claim 1 wherein the composition comprises 0.00001 to 50% by weight of the lipid component (i).

3. The cosmetic use of a composition according to claims 1 or 2 wherein the composition comprises 0.01 to 20% by weight of the lipid component (i).

4. The cosmetic use of a composition according to any one of claims 1 to 3 wherein the composition comprises 0.1 to 10% by weight of the lipid component (i).

5. The cosmetic use of a composition according to any preceding claim wherein the composition comprises 0.01 to 20% by weight of the organic acid component (ii).

6. The cosmetic use of a composition according to any one of claims 1 to 5 wherein the composition comprises 0.05 to 10% by weight of the organic acid component (ii).

7. The cosmetic use of a composition according to any preceding claim wherein the composition comprises 0.1 to 2% by weight of the organic acid component (ii).

8. The cosmetic use of a composition according to any preceding claim wherein the lipid component (i) is chosen from ceramides having the general structure (1) 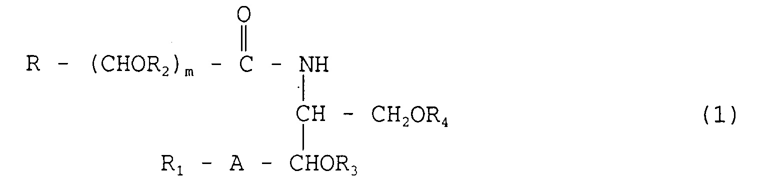 where A represents - CH₂ -; - CHOR₅ -; - CH=CH - or - CHOY -
R represents a linear or branched saturated or unsaturated, hydroxylated or non-hydroxylated aliphatic hydrocarbon group having from 1 to 49 carbon atoms or a subgroup (2).
Y - O - (CₐH_{b}) - (2)
R₁ represents a linear or branched, saturated or unsaturated, hydroxylated or non-hydroxylated aliphatic hydrocarbon group having from 8 to 28 carbon atoms;
R₂, R₃ and R₅ individually represent H, a phosphate residue or a sulphate residue;
R₄ represents H, a phosphate residue, a sulphate residue or a sugar residue;
a is an integer of from 7 to 49
b is an integer of from 10 to 98
m is 0 or 1
Y represents H or a residue of a C₁₄₋₂₂ fatty acid having the general structure (3) 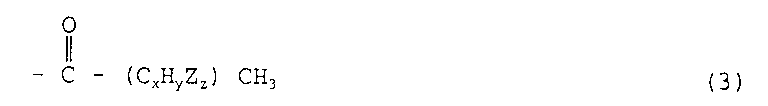 where
Z is - OH or an epoxy oxygen
x is an integer of from 12 to 20
y is an integer of from 20 to 40
and
z is O or an integer of from 1 to 4;
Pseudoceramides having the general structure (4) 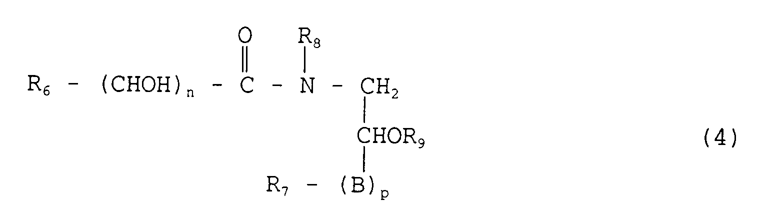 where
B represents - OCH₂ - or CHOH
R₆ represents a linear or branched, saturated or unsaturated, hydroxylated or non-hydroxylated aliphatic hydrocarbon group having from 1 to 49 carbon atoms or the subgroup (2).
R₇ represents a linear or branched, saturated or unsaturated, hydroxylated or non-hydroxylated hydrocarbon group having from 8 to 28 carbon atoms.
R₈ represents H, or a subgroup - (CH₂)_{c}COOH, where c is an integer of from 1 to 6, or a subgroup having the structure (5). 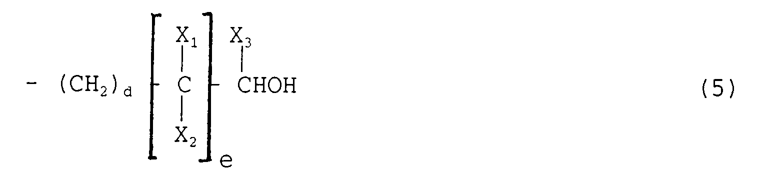 where X₁, X₂ and X₃ each individually represent H, a C₁₋₅ alkyl or a C₁₋₅ hydroxyalkyl;
d is 0 or an integer of from 1 to 4
e is 0 or 1
n is 0 or 1
and
p is 0 or 1;
R₉ is H, a phosphate residue, a sulphate residue or a sugar residue; and
mixtures thereof.

9. The cosmetic use of a composition according to any preceding claim wherein the organic acid component (ii) is chosen from α-hydroxy acids having the general structure (13): 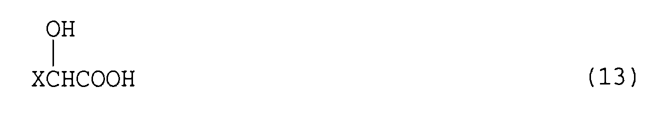 where
X is H - or CH₃(C_{f}H_{g})ₕ -
f is an integer of from 1 to 27,
g is an integer of from 2 to 54, and
h is 0 or 1.
hydroxymonocarboxylic acids having the general structure (14):
R₁₀(CR₁₁OH)_{q}(CH₂)ᵣCHOOH (14)
where R₁₀ and R₁₁ are individually H or an alkyl, aralkyl or aryl group of saturated, unsaturated, branched or unbranched chain or cyclic form having 1 to 25 carbon atoms
where
q is an integer of from 1 to 9, and
r is 0 or an integer of from 1 to 23;
hydroxydicarboxylic acids having the general structure (15) 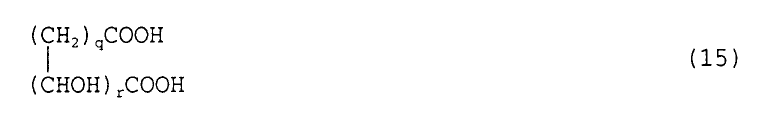 where
q is an integer of from 1 to 9, and
r is 0 or an integer of from 1 to 23;
hydroxycarboxylic acids having the general structure (16)
R₁₂(OH)_{q}(COOH)ₛ (16)
where q and s are individually an integer of from 1 to 9;
and R₁₂ is H, alkyl, aralkyl or aryl group of saturated or unsaturated, straight or branched chain or cyclic form having from 1 to 25 carbon atoms.
and mixtures thereof.

10. The cosmetic use of a composition according to any preceding claim wherein the organic acid component (ii) is chosen from 2-hydroxyoctanoic acid, glycolic acid, lactic acid and mixtures thereof.

11. The cosmetic use of a composition according to any preceding claim, wherein the composition additionaly comprises a fatty acid.

12. The cosmetic use of a composition according to any preceding claim wherein the composition additionally comprises sterol.

## Patentansprüche

1. Die kosmetische Verwendung einer Zusammensetzung für die örtliche Anwendung auf Haut, Haar oder Nägeln, umfassend:
(i) Eine Lipid-Komponente, ausgewählt aus Ceramiden, Pseudoceramiden und Mischungen derselben;
(ii) eine organische Säurekomponente, ausgewählt aus Hydroxycarbonsäuren, Ketocarbonsäuren und Mischungen derselben; und
(iii) einen kosmetisch annehmbaren Träger;
für die Behandlung und/oder Linderung von Zuständen der trockenen Haut.

2. Die kosmetische Verwendung einer Zusammensetzung nach Anspruch 1, worin die Zusammensetzung 0,00001 bis 50 Gewichtsprozent der Lipid-Komponente (i) enthält.

3. Die kosmetische Verwendung einer Zusammensetzung nach einem der Ansprüche 1 oder 2, worin die Zusammensetzung 0,01 bis 20 Gewichtsprozent der Lipid-Komponente (i) enthält.

4. Die kosmetische Verwendung einer Zusammensetzung nach einem der Ansprüche 1 bis 3, worin die Zusammensetzung 0,1 bis 10 Gewichtsprozent der Lipid-Komponente (i) enthält.

5. Die kosmetische Verwendung einer Zusammensetzung nach irgendeinem vorhergehenden Anspruch, worin die Zusammensetzung 0,01 bis 20 Gewichtsprozent der organischen Säurekomponente (ii) enthält.

6. Die kosmetische Verwendung einer Zusammensetzung nach irgendeinem der Ansprüche 1 bis 5, worin die Zusammensetzung 0,05 bis 10 Gewichtsprozent der organischen Säurekomponente (ii) enthält.

7. Die kosmetische Verwendung einer Zusammensetzung nach irgendeinem vorhergehenden Anspruch, worin die Zusammensetzung 0,1 bis 2 Gewichtsprozent der organischen Säurekomponente (ii) enthält.

8. Die kosmetische Verwendung einer Zusammensetzung nach irgendeinem vorhergehenden Anspruch, worin die Lipid-Komponente (i) ausgewählt ist aus Ceramiden der nachstehenden allgemeinen Struk tur (1) 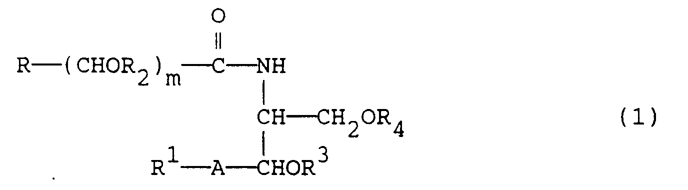 worin A -CH₂-; -CHOR⁵-; -CH=CH- oder -CHOY- bedeutet,
R eine lineare oder verzweigte, gesättigte oder ungesättigte, hydroxylierte oder nicht-hydroxylierte aliphatische Kohlenwasserstoff-Gruppe mit von 1 bis 49 Kohlenstoffatomen oder eine Untergruppe (2) der nachstehenden Formel
Y-O-(CₐH_{b})- (2)
ist.
R¹ bedeutet eine lineare oder verzweigte, gesättigte oder ungesättigte, hydroxylierte oder nicht-hydroxylierte aliphatische Kohlenwasserstoff-Gruppe mit von 8 bis 28 Kohlenstoffatomen;
R², R³ und R⁵ einzeln H, einen Phosphat- oder einen Sulfatrest bedeuten;
R⁴ ist H, ein Phosphatrest, ein Sulfatrest oder ein Zuckerrest;
a ist eine ganze Zahl von 7 bis 49
b ist eine ganze Zahl von 10 bis 98
m hat den Wert 0 oder 1;
Y ist H oder ein Rest von einer C₁₄₋₂₂-Fettsäure mit der allgemeinen Struktur (3) 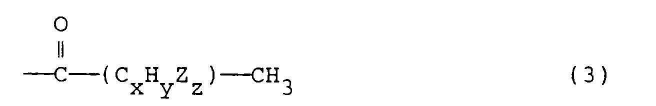 worin
Z -OH oder ein Epoxysauerstoff ist
x eine ganze Zahl von 12 bis 20 bedeutet
y eine ganze Zahl von 20 bis 40 ist und
z 0 ist oder eine ganze Zahl von 1 bis 4;
Pseudoceramide der allgemeinen Struktur (4) 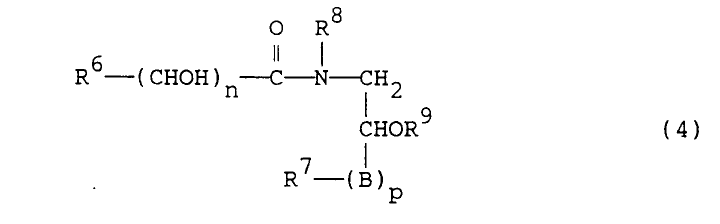 worin B -OCH₂- oder CHOH bedeutet.
R⁶ bedeutet eine lineare oder verzweigte, gesättigte oder ungesättigte, hydroxylierte oder nicht-hydroxylierte aliphatische Kohlenwasserstoff-Gruppe mit von 1 bis 49 Kohlenstoffatomen oder die Untergruppe (2).
R⁷ ist eine lineare oder verzweigte, gesättigte oder ungesättigte, hydroxylierte oder nicht-hydroxylierte Kohlenwasserstoff-Gruppe mit von 8 bis 28 Kohlenstoffatomen.
R⁸ bedeutet H oder eine Untergruppe -(CH₂)_{c}COOH, worin c eine ganze Zahl von 1 bis 6 ist, oder eine Untergruppe der nachfolgenden Struktur (5) 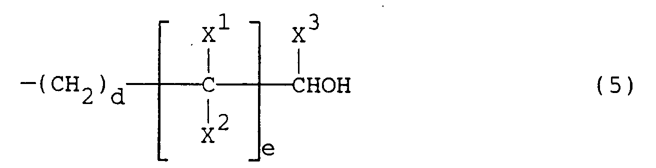 worin jeder Rest X¹, X² und X³ einzeln H, ein C₁₋₅-Alkyl oder ein C₁₋₅-Hydroxyalkyl ist;
d ist 0 oder eine ganze Zahl von 1 bis 4
e ist 0 oder 1
n ist 0 oder 1 und
p ist 0 oder 1;
R⁹ ist H, ein Phosphatrest, ein Sulfatrest oder ein Zuckerrest; und
Mischungen derselben.

9. Die kosmetische Verwendung einer Zusammensetzung nach irgendeinem der vorhergehenden Ansprüche, worin die organische Säurekomponente (ii) ausgewählt ist aus α-Hydroxysäuren der allgemeinen Struktur (13): 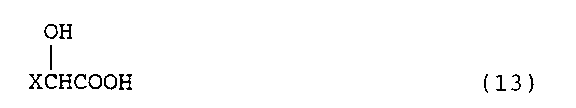 worin
X H- oder CH₃(C_{f}H_{g})ₕ ist,
f eine ganze Zahl von 1 bis 27 bedeutet,
g eine ganze Zahl von 2 bis 54 ist, und
h den Wert 0 oder 1 hat;
aus Hydroxymonocarbonsäuren der allgemeinen Struktur (14):
R¹⁰(CR¹¹OH)_{q}(CH₂)ᵣCOOH (14)
worin die Reste R¹⁰ und R¹¹, einzeln, H oder eine Alkyl-, Aralkyl- oder Arylgruppe von gesättigter, ungesättigter, verzweigter oder unverzweigter Ketten- oder cyclischer Form mit von 1 bis 25 Kohlenstoffatomen sind,
worin
q eine ganze Zahl von 1 bis 9 ist, und
r den Wert 0 hat oder eine ganze Zahl von 1 bis 23 ist;
aus Hydroxydicarbonsäuren der allgemeinen Struktur (15): 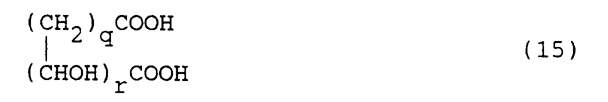 worin
q eine ganze Zahl von 1 bis 9 ist, und
r den Wert 0 hat oder eine ganze Zahl von 1 bis 23 ist;
aus Hydroxycarbonsäuren der allgemeinen Struktur (16)
R¹²(OH)_{q}(COOH)ₛ (16)
worin q und s, einzeln, eine ganze Zahl von 1 bis 9 sind; und R¹² H, eine Alkyl-, Aralkyl- oder Arylgruppe von gesättigter oder ungesättigter, gerader oder verzweigter Ketten- oder cyclischer Form mit von 1 bis 25 Kohlenstoffatomen ist;
und Mischungen derselben.

10. Die kosmetische Verwendung einer Zusammensetzung nach irgendeinem der vorhergehenden Ansprüche, worin die organische Säurekomponente (ii) aus 2-Hydroxyoctansäure, Glykolsäure, Milchsäure und Mischungen derselben, ausgewählt ist.

11. Die kosmetische Verwendung einer Zusammensetzung nach irgendeinem der vorhergehenden Ansprüche, worin die Zusammensetzung zusätzlich eine Fettsäure enthält.

12. Die kosmetische Verwendung einer Zusammensetzung nach irgendeinem der vorhergehenden Ansprüche, worin die Zusammensetzung zusätzlich Sterin enthält.

## Revendications

1. Utilisation cosmétique d'une composition destinée à être appliquée localement sur la peau, les cheveux ou les ongles, comprenant :
(i) un lipide choisi parmi les céramides, les pseudocéramides, et leurs mélanges ;
(ii) un acide organique choisi parmi les acides hydroxycarboxyliques, les acides cétocarboxyliques, et leurs mélanges ; et
(iii) un véhicule cosmétiquement acceptable ; pour le traitement et/ou l'atténuation des conditions de peau sèche.

2. Utilisation cosmétique d'une composition selon la revendication 1, dans laquelle la composition comprend de 0,00001 à 50%, en poids, du lipide (i).

3. Utilisation cosmétique d'une composition selon les revendications 1 ou 2, dans laquelle la composition comprend de 0,01 à 20%, en poids, du lipide (i).

4. Utilisation cosmétique d'une composition selon l'une quelconque des revendications 1 à 3, dans laquelle la composition comprend de 0,1 à 10%, en poids, du lipide (i).

5. Utilisation cosmétique d'une composition selon l'une quelconque des revendications précédentes, dans laquelle la composition comprend de 0,01 à 20%, en poids, de l'acide organique (ii).

6. Utilisation cosmétique d'une composition selon l'une quelconque des revendications 1 à 5, dans laquelle la composition comprend de 0,05 à 10%, en poids, de l'acide organique (ii).

7. Utilisation cosmétique d'une composition selon l'une quelconque des revendications précédentes, dans laquelle la composition comprend de 0,1 à 2%, en poids, de l'acide organique (ii).

8. Utilisation cosmétique d'une composition selon l'une quelconque des revendications précédentes, dans laquelle le lipide (i) est choisi parmi les céramides de structure générale (1) 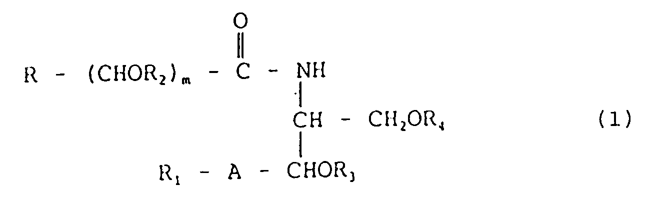 où A représente - CH₂ - ; - CHOR₅ - ; - CH=CH - ou - CHOY -
R représente un groupe hydrocarboné aliphatique linéaire ou ramifié, saturé ou insaturé, hydroxylé ou non-hydroxylé, ayant de 1 à 49 atomes de carbone ou un sous-groupe (2).
Y - O - (CₐH_{b}) - (2)
R₁ représente un groupe hydrocarboné aliphatique linéaire ou ramifié, saturé ou insaturé, hydroxylé ou non-hydroxylé, ayant de 8 à 28 atomes de carbone ;
R₂, R₃ et R₅ représentent, individuellement, H, un résidu de phosphate ou un résidu de sulfate ;
R₄ représente H, un résidu de phosphate, un résidu de sulfate ou un résidu de sucre ;
a est un entier de 7 à 49
b est un entier de 10 à 98
m est 0 ou 1
Y représente H ou un résidu d'un acide gras en C₁₄₋₂₂ de structure générale (3) 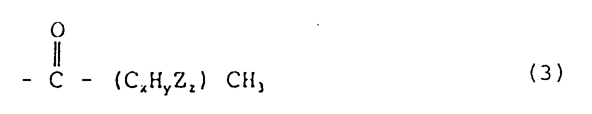 où
Z est -OH ou un atome d'oxygène époxy
x est un entier de 12 à 20
y est un entier de 20 à 40
et
z est 0 ou un entier de 1 à 4 ;
les pseudocéramides de structure générale (4) 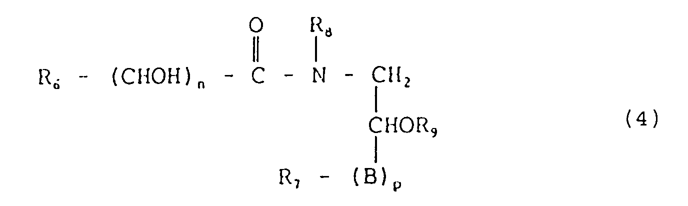 où B représente - OCH₂ - ou CHOH
R₆ représente un groupe hydrocarboné aliphatique linéaire ou ramifié, saturé ou insaturé, hydroxylé ou non-hydroxylé, ayant de 1 à 49 atomes de carbone ou le sous-groupe (2).
R₇ représente un groupe hydrocarboné linéaire ou ramifié, saturé ou insaturé, hydroxylé ou non-hydroxylé, ayant de 8 à 28 atomes de carbone.
R₈ représente H, ou un sous-groupe -(CH₂)_{c}COOH, où c est un entier de 1 à 6, ou un sous-groupe de structure (5) 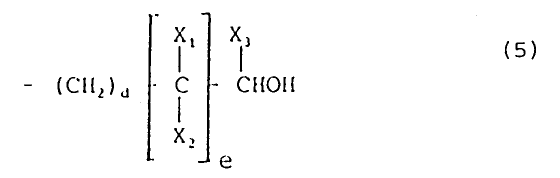 où X₁, X₂ et X₃ représentent, chacun individuellement, H, un alkyle en C₁₋₅ ou un hydroxyalkyle en C₁₋₅ ;
d est 0 ou un entier de 1 à 4
e est 0 ou 1
n est 0 ou 1
et
p est 0 ou 1 :
R₉ représente H, un résidu de phosphate, un résidu de sulfate ou un résidu de sucre, et leurs mélanges.

9. Utilisation cosmétique d'une composition selon l'une quelconque des revendications précédentes, dans laquelle l'acide organique (ii) est choisi parmi les acides α-hydroxyliques de structure générale (13) : 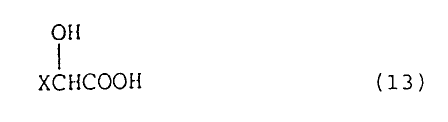 où
X est H - ou CH₃(C_{f}H_{g})ₕ -
f est un entier de 1 à 27,
g est un entier de 2 à 54, et h est 0 ou 1
les acides hydroxymonocarboxyliques de structure générale (14) :
R₁₀(CR₁₁OH)_{q}(CH₂)ᵣCOOH (14)
où R₁₀ et R₁₁ sont, individuellement, H ou un groupe alkyle, aralkyle ou aryle de forme cyclique ou à chaîne ramifiée ou non-ramifiée, saturé ou insaturé, ayant de 1 à 25 atomes de carbone
où
q est un entier de 1 à 9, et
r est 0 ou un entier de 1 à 23 ;
les acides hydroxydicarboxyliques de structure générale (15) 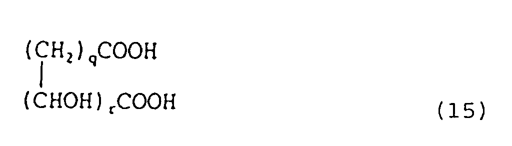 où
q est un entier de 1 à 9, et
r est 0 ou un entier de 1 à 23 ;
les acides hydroxycarboxyliques de structure générale (16)
R₁₂(OH)_{q}(COOH)ₛ (16)
où q et s sont, individuellement, des entiers de 1 à 9 ;
et R₁₂ est H, un groupe alkyle, aralkyle ou aryle de forme cyclique ou à chaîne ramifiée ou non-ramifiée, saturé ou insaturé, ayant de 1 à 25 atomes de carbone ; et leurs mélanges.

10. Utilisation cosmétique d'une composition selon l'une quelconque des revendications précédentes, dans laquelle l'acide organique (ii) est choisi parmi l'acide 2-hydroxyoctanoïque, l'acide glycolique, l'acide lactique, et leurs mélanges.

11. Utilisation cosmétique d'une composition selon l'une quelconque des revendications précédentes, dans laquelle la composition comprend en plus un acide gras.

12. Utilisation cosmétique d'une composition selon l'une quelconque des revendications précédentes, dans laquelle la composition comprend en plus un stérol.
